# EUROPEAN PATENT SPECIFICATION

(11) **EP 1 728 480 B1**
(45) Date of publication and mention of the grant of the patent: **09.04.2014**
(21) Application number: 06252829.4
(22) Date of filing: 01.06.2006
(51) Int. Cl.: A61B 17/68, A61B 17/80

(54) **Scapholunate disassociation repair system**
System zur Reparatur einer skapholunären Dissoziation
Système de réparation d'une dissociation scapholunaire

(30) Priority: 02.06.2005 US 686854 P
(43) Date of publication of application: 06.12.2006
(73) Proprietor: Biomet C.V., Gibraltar (GI)
(72) Inventor: Orbay, Jorge L., Miami, FL 33156 (US); Nunez, Jose Antonio, Miami, FL 33194 (US); Castaneda, Javier E., Miami, FL 33186 (US)
(74) Representative: Giles, Ashley Simon

(56) References cited:
- EP-A- 1 273 269
- EP-A2- 1 690 499
- WO-A1-82/01645
- WO-A2-2006/122218
- DE-A1- 4 018 273
- US-A- 5 558 674
- US-A- 5 827 285
- US-A- 5 853 413
- US-A- 5 891 168
- US-A1- 2005 187 554
- US-B1- 6 221 073
- US-B1- 6 663 633

## Description

This invention relates broadly to surgery. More particularly, this invention relates to orthopaedic implants for the repair of the scapholunate ligament.

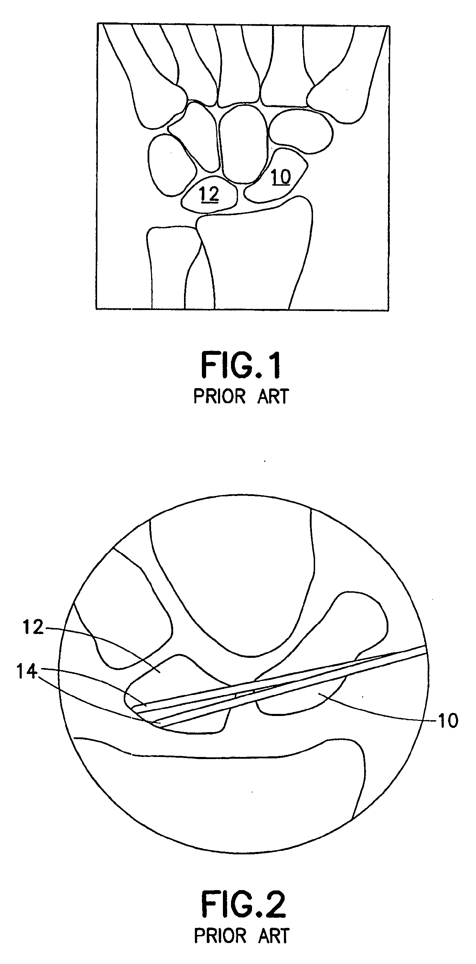

The rupture of the scapholunate ligament is a well-known clinical problem and can result from trauma or attrition. The scapholunate ligament ties together the scaphoid and lunate bones and is a keystone for stability of the carpal bones. Referring to the accompanying Fig. 1, malfunction of the scapholunate ligament often results in severe carpal dissociation (with separation of the scaphoid 10 and lunate 12) and derangement of the carpal kinematics.

Historically, attempts to surgically repair or reconstruct the ruptured scapholunate ligament have been unsuccessful. The reason for this is not well understood. One hypothesis is that (i) the ligament carries very high forces during normal use; (ii) the ligament is very short (only 2 to 3 mm in length) and therefore any small deformation is a very high percentage of the entire length of the whole ligament resulting in a large amount of strain; and (iii) there has been no adequate form of immobilization for this small joint.

Referring to Fig. 2, conventional treatment for scapholunate disassociation repair utilizes K-wires 14 inserted laterally through the scaphoid 10 and into the lunate 12 to immobilize the joint and permit ligament healing. However, the K-wires 14 tend to move, do not provide sufficient immobilization at the required location, and need to be removed prior to completion of the prolonged healing process.

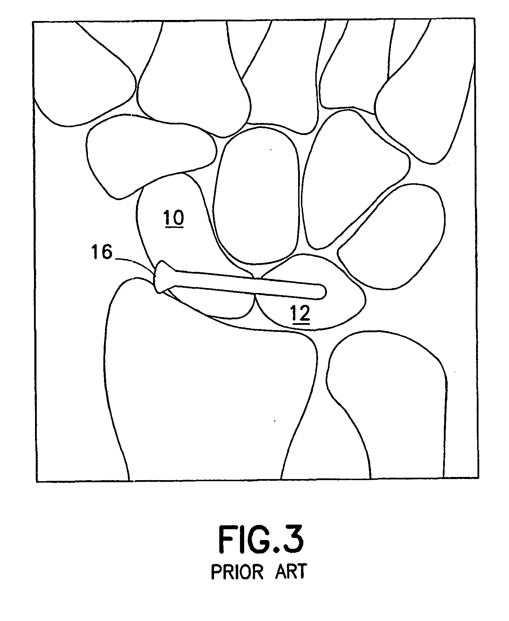

Referring to Fig. 3, screws 16 have also been used for temporary scapholunate fixation. Like K-wires, the screw is inserted in the medial-lateral plane, through the scaphoid 10 and lunate 12 to provide immobilization during ligament healing. This technique also has a high failure rate due to screw loosening and backing out.

There are many soft tissue techniques for repair of the scapholunate ligament, each making an attempt to suture the ligament or reconstruct it by bringing in new ligament tissue. They all need a form of temporary fixation of the scapholunate joint for success. Little progress has been made in providing the surgeon with a better form of temporary fixation.

US-5558674 discloses devices and methods for posterior spinal fixation. Additionally, the devices and methods may be applied to general bone fixation and scull fracture repair. A fixing plate includes a plurality of fixation holes for receiving screws passing into bone.

This document discloses the technical features of the preamble of claim 1.

US-6221073 discloses a wrist fusion apparatus for attachment to and positioning at least one carpus bone relative to a radius bone without an attachment to a metacarpal bone. The apparatus includes an elongated plate having a proximal portion for positioning an attachment to a radius bone and a distal portion for positioning over an attachment to at least one carpus bone. The plate includes fastener holes defined therethrough for receiving bones fasteneres.

US-6663633 discloses an orthopedic fixation and reduction system comprising a helical fixation element biased to a predetermined pitch and a generally helical insertion element dimension to admit at least a distal portion of the fixation element into a lumen thereof. The orthopedic fixation system can be used for stabilising two sections of a bone in spaced apart relation.

EP-1273269 discloses a bone screw having a flexible shaft which prevents relative movement in the direction of tension. The screw may be positioned in a bore extending through a scaphoid bone and a lunate bone in order to repair a ruptured scapholunate ligament.

The invention seeks to provide a system for scapholunate ligament repair which provides satisfactory fixation.

According to a first aspect the present invention provides a scapholunate disassociation repair system, comprising: a first plate sized for placement on a scaphoid bone, said first plate including at least one fixed angle hole therein; a second plate sized for placement on a lunate bone, said second plate including at least one fixed angle hole therein; fixed angle fasteners for insertion through said fixed angle holes in said first and second plates for coupling said first and second plates to said scaphoid bone and said lunate bone respectively; and an element joining said first and second plates; characterised in that the element is flexible and moveably joins said first and second plates together such that when said first and second plates are coupled to surfaces of scaphoid and lunate bones, the scaphoid and lunate bones can translate and rotate relative to one another; wherein at least one of the ends of the element is moveable relative to at least one plate then lockable in position such that the element is adjustable in length to space the scaphoid and lunate bones apart a dorsal scapholunate interval.

The two plates may be coupled by a metal or polymeric flexible element. The flexible element acts like an artificial ligament and permits more normal motion of the wrist bones while maintaining the scapholunate interval at its correct anatomical dimension.

The system of the invention can be used in a method of scapholunate repair, which comprises the scaphoid and lunate bone together in a manner which maintains the dorsal scapholunate interval between the scaphoid and lunate bones while permitting movement of the scapholunate joint.

Embodiments of the invention will now be described by way of example with reference to the accompanying drawings, in which:
PRIOR ART FIG. 1 is an X-ray volar view of a right hand showing a scapholunate dissociation as a result of a rupture of the scapholunate ligament;
PRIOR ART FIG. 2 is a fluoroscopic volar view of a right hand showing the use of K-wires to treat a scapholunate dissociation;
PRIOR ART FIG. 3 is an X-ray volar view of a left hand showing the use of a bone screw to treat a scapholunate dissociation;
FIG. 4 is a top view of a plate for a scapholunate disassociation repair system;
FIG. 5 is a proximal-distal view of the plate of FIG. 4;
FIG. 6A is a constructed X-ray volar view of a right hand showing a scapholunate disassociation repair system including the plate of FIG. 4 implanted in a wrist;
FIG. 6B is a constructed X-ray medial view of a right hand showing a scapholunate disassociation repair system including the plate of FIG. 4 implanted in a wrist;
FIG. 7 is a top view of an alternative plate of a scapholunate disassociation repair system;
FIG. 8 is a proximal-distal view of the plate of FIG. 7;
FIG. 9 is a constructed X-ray volar view showing an implanted scapholunate disassociation repair system:
FIG. 10 is a dorsal view of a scapholunate disassociation repair system according to an embodiment of the invention;
FIG. 11 is a dorsal view of a scapholunate disassociation repair system;
FIG. 12 is a constructed X-ray volar view showing an implanted scapholunate disassociation repair system; and
FIG. 13 is a schematic view of an implanted scapholunate disassociation repair system.

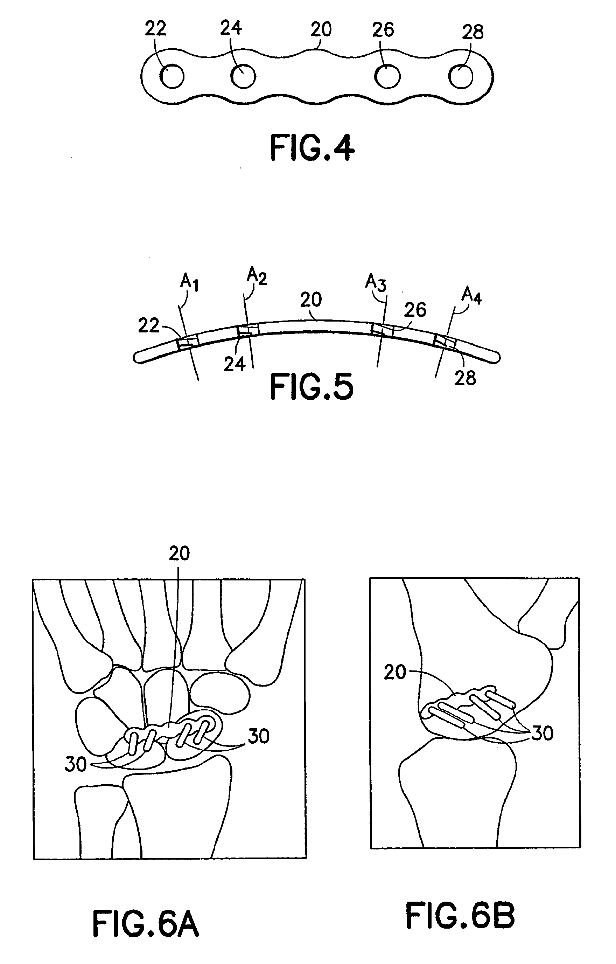

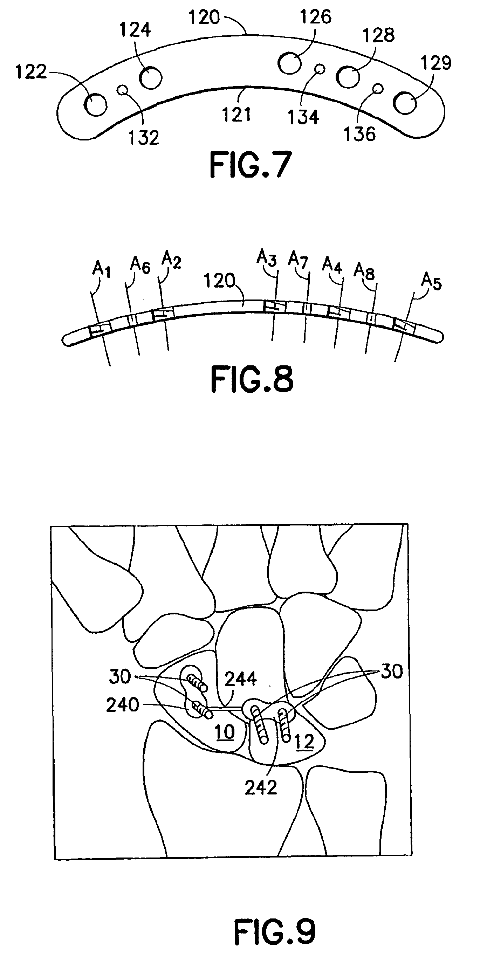

Referring to the drawings, Figs. 4 and 5 show a small contoured fixed angle plate 20 is provided for the temporary fixation of the scapholunate interval between the scaphoid and lunate carpal bones. The plate 20 is preferably curved for placement on the dorsal anatomy of the scaphoid and lunate bones. That is, it is curved both concavely at the bone contacting surface and side-to-side, as described further below. The plate 20 includes a plurality of holes 22, 24, 26, 28 for receiving fasteners 30 (Figs. 6 and 7) at a fixed angle relative to the plate. To achieve fixed angle fixation holes 22-28 are preferably threaded, and the heads of the fasteners are threaded to engage the holes. Other means to fix the fasteners relative to the plate may also be used. The shafts of the fasteners are also preferably threaded for rigid engagement with the carpal bones. Holes 22, 24 receive fasteners for insertion into the scaphoid, while holes 26, 28 receive fasteners for insertion into the lunate. Axes A1, A2 through holes 22, 24 are preferably obliquely oriented relative to axes A3, A4, so that the fasteners conform to the scaphoid and lunate anatomy. In addition, axes A 1 and A2 may be oblique relative to each other, and axes A3 and A4 may also be oblique relative to each other. The plate maintains an optimum dorsal scapholunate interval during healing; i.e., preferably approximately 4.2 mm, in accord with the average dorsal scapholunate interval, but the plate can be provided in sizes to accommodate other common scapholunate intervals, e.g., in the range from 2.3 to 6.3 mm.

The plate 20 and fasteners 30 are implanted on a dorsal side of the scaphoid and lunate bones preferably for at least a semi-permanent amount of time (preferably at least nine months), allowing complete healing and remodeling of the scapholunate ligament. The system creates a temporary or reversible arthrodesis which allows almost full function of the hand without cast support. The system can optionally be removed post-healing to allow full mobility of the wrist.

Turning now to Figs. 7 and 8, a plate 120 (substantially similar to plate 20) for a scapholunate disassociation repair system is shown. The plate 120 is curved side-to-side (see at distal side 121), preferably along a radius of 30 to 35 mm, to fit the scaphoid and lunate bones better. Two fastener holes 122, 124 are provided for the lunate, while three fastener holes 126, 128, 129 are provided for the relatively larger scaphoid. Smaller non-threaded alignment holes 132, 134, 136 sized to receive K-wires in a fixed angle orientation are provided between the fastener holes. K-wires can be inserted through the alignment holes 132, 134, 136 to provide temporary fixation and fluoroscopic visualization of the plate and intended approximate trajectories of the fasteners. As the axes A6, A7 and A8 through the respective alignment holes are centred between the axes of the surrounding fastener holes A1, A2, A3, A4 and A5, K-wires inserted through them provide a good indication of whether the fasteners will be properly inserted prior to implantation. If visualization reveals that intended implantation requires modification, the K-wires can easily be removed and the plate repositioned until the location is appropriate. Removal of the K-wires creates far less tissue disruption that removal of an implanted fastener.

Referring to Fig. 9, the system for scapholunate disassociation repair includes two small plates, one plate 240 for the scaphoid and the other plate 242 for the lunate. Each plate 240, 242 is attached to its respective bone using fasteners 30 as described above. The two plates 240, 242 are attached by a flexible element 244. The flexible element 244 may be, by way of example, a metal braided cable, metal monofilament wire, polymeric filament, polymeric band, polymeric braid, or a combination thereof which is fixedly coupled to each plate via welding, brazing, soldering, bonding, crimping, tying, a set screw, or any other suitable means. Other suitable flexible elements may similarly be used. The flexible element 244 functions like an artificial ligament to permits more normal motion of the wrist bones while maintaining the scapholunate interval at its correct anatomical dimension. The scapholunate joint does have some physiologic motion which is quite complex and has a center of rotation outside of the body of either the scaphoid and lunate. This motion includes translation and rotation and can only be allowed by a flexible element 244 that has the insertion points of the scapholunate ligament.

The system of Fig. 9 accurately reproduces the insertion sites of the scapholunate ligament and allows for very normal kinematics while the healing process occurs. Like all the other examples, the elements of the system can be permanent or removed after ligament healing, usually nine months post-operative.

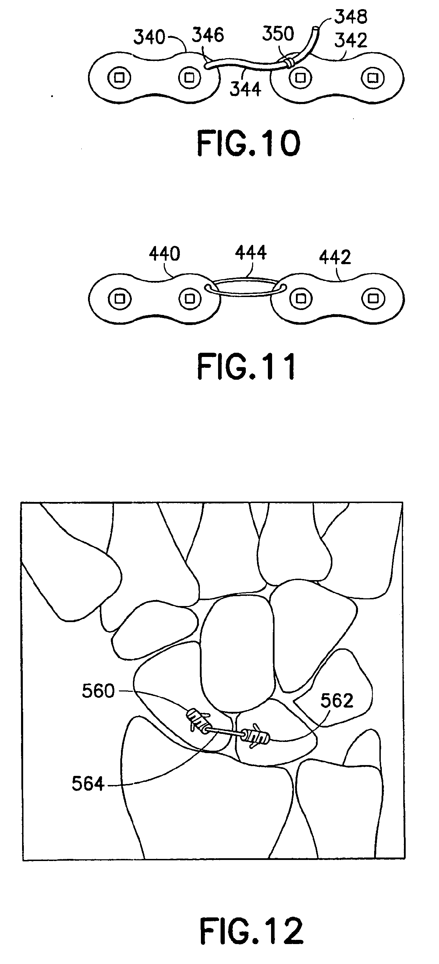

Turning now to Fig. 10, an embodiment of the system is substantially the same as the system described with respect to Fig. 9. In this embodiment of Fig. 10, one end 346 of the flexible element 344 is fixedly coupled to one plate, e.g., 340, while the other end (free end) 348 may be moved relative to the second plate, e.g., 342 during implantation. Then, once both the plates are implanted on the scaphoid and lunate, and the scaphoid and lunate are anatomically positioned, the free end 348 is pulled and the second plate 342 is locked relative to the flexible element to maintain the scapholunate interval. The flexible element and plate are locked relative to each other using, e.g., deformation crimping of a band 350 through which the flexible element 344 extends or rotation of a set screw which compresses the flexible element. In this manner, the procedure is facilitated by providing additional slack between the plates during plate implantation, but the ability to correct the anatomical derangement is not compromised.

In an alternate embodiment, both plates may be movable relative to the flexible element and then lockable in position. In yet another alternate embodiment, the plates may be discretely coupled to the scaphoid and lunate (without any flexible element extending between them), and the flexible element may thereafter be introduced and coupled to the plates.

Turning now to Fig. 11, the plates 440, 442 may be movably joined to a relatively rigid link 444 (or other substantially rigid member) which spaces the plates at the appropriate scapholunate interval and allows the plates to move in the desired degrees of freedom.

Turning now to Fig. 12, the system for scapholunate disassociation repair is plateless, including two bone anchors, one anchor 560 on the scaphoid and the other anchor 562 on the lunate, joined by a flexible element 564. The fifth embodiment functions similarly to the third embodiment and has the same advantages. Similarly, the anchors and flexible element may be configured such that the at least one anchor is movable relative to an end of the flexible element (to facilitate implantation), moved thereto upon alignment of the anatomy, and then locked relative thereto to maintain the scapholunate interval. It is appreciated that the plateless embodiment lends itself to an arthroscopic approach to scapholunate disassociation repair. Alternatively, the bone anchors may be movably joined to a non-flexible central element, as discussed above with respect to Figure 11.

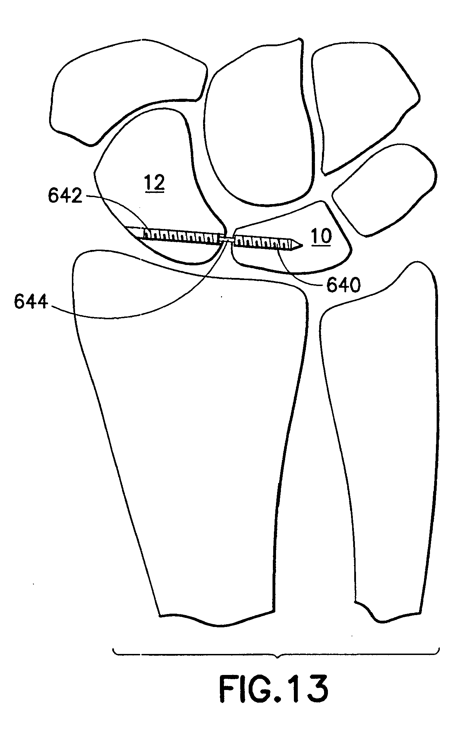

Referring now to Fig. 13, another system for scapholunate disassociation repair is shown. The system is adapted for percutaneous repair, including first and second screw portions 640, 642, and a flexible member 644 therebetween. The first and second screw portions 640, 642 are initially coupled together. The first screw portion 640 is percutaneously introduced through the lunate bone and into the scaphoid bone 10, and the second screw portion 642 is percutaneously introduced into the lunate bone 12. The first and second screw portions are then decoupled. The flexible member 644 extends between the screw portions 640, 642 and thus the two bones 10, 12 to hold the scaphoid and lunate in relation at the appropriate scapholunate interval, while permitting the desired degrees of freedom between the bones.

While fixed angle threaded peg fasteners have been described for use with the plate, it will be appreciated that other fasteners including smooth shaft pegs, fixed angle fasteners which do not require a threaded head engaging a threaded hole, and even non-fixed angle fasteners may be used with the plates described herein, such plates being modified as necessary for use with such fasteners.

## Claims

1. A scapholunate disassociation repair system, comprising:
a first plate (340) sized for placement on a scaphoid bone, said first plate (340) including at least one fixed angle hole therein;
a second plate (342) sized for placement on a lunate bone, said second plate (342) including at least one fixed angle hole therein;
fixed angle fasteners (30) for insertion through said fixed angle holes in said first and second plates (340, 342) for coupling said first and second plates (340, 342) to said scaphoid bone and said lunate bone respectively; and
an element (344) joining said first and second plates (344);
**characterised in that** the element (344) is flexible and moveably joins said first and second plates (340, 342) together such that when said first and second plates (340, 342) are coupled to surfaces of scaphoid and lunate bones, the scaphoid and lunate bones can translate and rotate relative to one another;
wherein at least one of the ends of the element (344) is moveable relative to at least one plate then lockable in position such that the element (344) is adjustable in length to space the scaphoid and lunate bones apart a dorsal scapholunate interval.

2. A system according to claim 1, wherein said plates (340, 342) are arranged to be attached to dorsal surfaces of the scaphoid and lunate bones.

3. A system according to claim 1, in which the first plate (340) includes two fixed angle holes each defining a central axis, said central axes of said two holes being obliquely angled relative to each other.

4. A system according to claim 3, in which the second plate (342) includes two fixed angle holes each defining a central axis, said central axes of said two holes of said second plate (342) being obliquely angled relative to each other.

## Patentansprüche

1. Reparatursystem für eine skapholunäre Dissoziation, umfassend:
eine erste Platte (340) mit den entsprechenden Abmessungen für das Platzieren auf dem Kahnbein, wobei in der ersten Platte (340) mindestens ein winkelstabiles Loch ist,
eine zweite Platte (342) mit den entsprechenden Abmessungen für das Platzieren auf dem Mondbein, wobei in der zweiten Platte (342) mindestens ein winkelstabiles Loch ist,
winkelstabile Befestigungsmittel (30) zum Einsetzen durch die winkelstabilen Löcher in der ersten und zweiten Platte (340, 342), so dass die erste und die zweite Platte (340, 342) am Kahnbein bzw. Mondbein befestigt werden, und
ein Element (344), das die erste und zweite Platte miteinander verbindet (344),
**dadurch gekennzeichnet, dass** das Element (344) flexibel ist und die erste und zweite Platte (340, 342) derart miteinander verbindet, dass nach dem Befestigen der ersten und zweiten Platte (340, 342) an den Oberflächen des Kahnbeins bzw. Mondbeins diese Knochen relativ zueinander verschiebbar und drehbar sind,
wobei mindestens ein Ende des Elements (344) in Bezug auf mindestens eine Platte bewegbar und dann in der Position verriegelbar ist, so dass das Element (344) in der Länge derart eingestellt werden kann, dass es das Kahn- und das Mondbein um einen dorsalen skapholunären Spalt voneinander beabstandet.

2. System nach Anspruch 1, wobei die Platten (340, 342) für das Anbringen an dorsale Oberflächen des Kahnbeins bzw. Mondbeins ausgelegt sind.

3. System nach Anspruch 1, wobei die erste Platte (340) zwei winkelstabile Löcher enthält, die jeweils eine Mittelachse festlegen, wobei die Mittelachsen der beiden Löcher schräg zueinander verlaufen.

4. System nach Anspruch 3, wobei die zweite Platte (342) zwei winkelstabile Löcher enthält, die jeweils eine Mittelachse festlegen, wobei die Mittelachsen der beiden Löcher der zweiten Platte (342) schräg zueinander verlaufen.

## Revendications

1. Système de réparation d'une dissociation scapho-lunaire, comprenant :
une première plaque (340) dimensionnée pour être placée sur un os scaphoïde, ladite première plaque (340) incluant au moins un trou d'angle fixe dans celle-ci ;
une deuxième plaque (342) dimensionnée pour être placée sur un semi-lunaire, ladite deuxième plaque (342) incluant au moins un trou d'angle fixe dans celle-ci ;
des attaches d'angle fixe (30) pour l'insertion à travers lesdits trous d'angle fixes dans lesdites première et deuxième plaques (340, 342) pour le couplage desdites première et deuxième plaques (340, 342) audit os scaphoïde et audit semi-lunaire, respectivement ; et
un élément (344) reliant lesdites première et deuxième plaques (344) ;
**caractérisé en ce que** l'élément (344) est flexible et relie d'une manière mobile lesdites première et deuxième plaques (340, 342) ensemble de sorte que lorsque lesdites première et deuxième plaques (340, 342) sont couplées à des surfaces de l'os scaphoïde et au semi-lunaire, l'os scaphoïde et le semi-lunaire peuvent translater et tourner l'un relativement à l'autre,
où au moins une des extrémités de l'élément (344) est déplaçable relativement à au moins une plaque, peut ensuite être verrouillée en position de sorte que l'élément (344) est ajustable en longueur pour espacer l'os scaphoïde et le semi-lunaire à un intervalle scapho-lunaire dorsal.

2. Système selon la revendication 1, dans lequel lesdites plaques (340, 342) sont agencées pour être fixées à des surfaces dorsales de l'os scaphoïde et du semi-lunaire.

3. Système selon la revendication 1, dans lequel la première plaque (340) comprend deux trous d'angle fixes, chacun définissant un axe central, lesdits axes centraux desdits deux trous présentant un angle oblique l'un relativement à l'autre.

4. Système selon la revendication 3, dans lequel la deuxième plaque (342) comprend deux trous d'angle fixes, chacun définissant un axe central, lesdits axes centraux desdits deux trous de ladite deuxième plaque (342) présentant un angle oblique l'un relativement à l'autre.
